# EUROPÄISCHE PATENTSCHRIFT

(11) **EP 3 231 520 B1**
(45) Veröffentlichungstag und Bekanntmachung des Hinweises auf die Patenterteilung: **27.06.2018**
(21) Anmeldenummer: 17157719.0
(22) Anmeldetag: 23.02.2017
(51) Int. Cl.: B08B 3/08, B65B 55/10, B65B 57/08, B65B 1/04, B65B 7/16, B65B 31/04, B65B 1/00, B65B 41/00, B65B 43/54, B65B 43/52, B65B 25/00, B65B 43/59, B65B 55/04, B65B 55/24, B65B 65/02, B65B 3/00, B65B 3/04, B65B 7/28, B65D 85/72

(54) **VORRICHTUNG ZUM ABFÜLLEN VON FLÜSSIGEN LEBENSMITTELN**
DEVICE FOR FILLING LIQUID FOODSTUFF
DISPOSITIF DE L'EMBOUTILLAGE DES ALIMENTS LIQUIDES

(30) Priorität: 30.03.2016 DE 102016105780
(43) Veröffentlichungstag der Anmeldung: 18.10.2017
(73) Patentinhaber: Hamba Filltec GmbH & Co. KG, 66130 Saarbrücken (DE)
(72) Erfinder: Daniel, Jan Simon, 66287 Quierschied (DE)
(74) Vertreter: Ostriga Sonnet Wirths & Vorwerk

(56) Entgegenhaltungen:
- DE-A1- 2 509 611
- DE-A1- 3 931 672
- DE-A1- 10 309 259
- US-A- 4 537 749

## Beschreibung

Die Erfindung betrifft eine Vorrichtung zum Abfüllen von flüssigen bis pastösen Lebensmitteln in vorgefertigte Einzelbehälter, die einen Tragrand aufweisen, mit Tragelementen, die mittels eines Antriebs endlos durch die Vorrichtung geführt sind, Aufnahmeöffnungen für die Einzelbehälter ausbilden und eine der Schwerkraft abgewandte Oberseite aufweisen, auf welcher der Tragrand der in den Aufnahmeöffnungen einsitzenden Einzelbehälter aufzuliegen in der Lage ist, mit Arbeitsstationen, in deren Wirkbereich die Tragelemente auf ihrem endlosen Umlauf in der Vorrichtung gelangen, mit wenigstens einer als Sterilisationseinrichtung ausgebildeten Arbeitsstation, welche der Entkeimung von Tragelementen und Einzelbehältern dient, mit einer Anhebevorrichtung als Teil der Sterilisationseinrichtung, welche in den Aufnahmeöffnungen einsitzende und mit dem Tragrand auf der Oberseite aufliegende Einzelbehälter zur Sterilisation spaltbildend anzuheben in der Lage ist, mit einer Steuerung, die zumindest der Steuerung der Anhebevorrichtung dient.

Gattungsgemäße Abfüllvorrichtungen sind beispielsweise aus EP 1 134 182 A1 oder EP 2 527 260 A1 der Anmelderin bekannt. Beide Vorrichtungen verfügen über eine Vielzahl von Tragelementen, die ihrerseits eine Vielzahl von Aufnahmeöffnungen für Einzelbehälter aufweisen. Diese Tragelemente werden über ein Obertrum und ein Untertrum endlos umlaufend durch die Abfüllanlage geführt. Dabei sind die Tragelemente zumindest in demjenigen Trum, welches über die die Behälter behandelnden Arbeitsstationen verfügt, unmittelbar aneinander angeordnet. Auf diese Weise sind die Aufnahmeöffnungen in diesem Trum in Durchlaufrichtung hintereinander zu Reihen und quer zur Durchlaufrichtung nebeneinander in Bahnen angeordnet.

Die Arbeitsstationen der Abfüllvorrichtung führen vorgefertigte Einzelbecher zu, setzen diese in Aufnahmeöffnungen ein, führen eine Sterilisation der Becher und Tragelemente durch, Befüllen die Einzelbehälter sodann mit Lebensmitteln, wie beispielsweise Joghurt, einem Getränk, einem Streichfett, etc.. Sodann werden die Einzelbecher in einer Siegelstation mit einer Siegelfolie belegt und verschlossen, ggf. einer Dichtigkeitsprüfung unterzogen und schließlich zu Gebinden zusammengefasst und abgepackt. Soweit leicht verderbliche Lebensmittel, wie beispielsweise Joghurt, abgefüllt werden, ist eine solche Abfülleinrichtung zumindest in einem Sterilbereich eingehaust. In diese Einhausung wird Sterilluft eingeblasen, so dass dort ein das Eindringen von Keimen über die Luft verhindernder Überdruck besteht.

Der wesentliche Unterschied zwischen den beiden als Stand der Technik angeführten Abfüllvorrichtungen liegt in der Antriebsart. Beim erstgenannten Stand der Technik sind die Zellenbretter auf einem Endlosfördermittel, in der Regel eine Kette, befestigt und werden mittels dieser umlaufend durch die Vorrichtung transportiert. Der zweitgenannte Stand der Technik verzichtet auf das Endlosantriebsmittel, welches unter bestimmten Bedingungen gravierende Nachteile mit sich bringen kann.

Grundsätzlich ist beim Abfüllen von Nahrungsmitteln das Einhalten hoher Hygienestandards erforderlich, wobei diese sich je nach Art des Produktes in gewissem Rahmen voneinander unterscheiden. Die vorliegende Erfindung betrifft eine Vorrichtung zum Abfüllen von Nahrungsmitteln mit geringem Säuregehalt in Einzelbecher, wie beispielsweise zum Abfüllen von Soßen für Fertiggerichte, Snacks oder Fingerfood. Solche Produkte sind in der Regel sehr empfindlich, so dass bereits geringe Keimbelastungen, die beispielsweise bei Produkten mit höherem Säuregehalt unproblematisch sind, zu einem schnellen Verderben der Lebensmittel führen.

Um die Keimbelastung ausreichend gering zu halten, werden an die Sterilisationseinrichtung hohe Anforderungen gestellt, zumal der hohe Vorzugstakt der Tragelemente lediglich eine geringe Verweildauer in der Sterilisationseinrichtung ermöglicht.
Mit Einzelbechern befüllte Tragelemente werden deshalb in die Sterilisationseinrichtung eingebracht und mit einem feinen Peroxidnebel belegt. Hierbei werden die Einzelbecher geringfügig angehoben, so dass sich ein Spalt zwischen dem Tragrand des Behälters und der Oberseite des Tragelementes bildet. Durch diesen Spalt dringt der sterilisierende Peroxidnebel auch in die sonst abgedeckten Oberflächenbereiche des Tragelementes.
Es besteht jedoch auch die Notwendigkeit, behälterlose Tragelemente ausreichend zu sterilisieren, da beim Anfahren oder Auslaufen eines Produktionszykluses, bei Störungen in der Behälterzuführung oder der Behälterversiegelung behälterlose Zellenbretter in den Sterilbereich der Anlage einlaufen können. Dieser Sterilbereich erstreckt sich von der Behältersterilisation bis in den Bereich der Behälterversiegelung.

Andere gattungsgemäße Vorrichtungen sind Gegenstand der DE 103 09 259 A1 und der DE 39 31 672 A1.

Aufgabe der Erfindung ist es deshalb, eine Vorrichtung gattungsgemäßer Art zu schaffen, dessen Sterilisationseinrichtung auch behälterlose Zellenbretter ausreichend keimfrei hält.
Gelöst wird die Aufgabe von einer Vorrichtung mit den Merkmalen des Anspruches 1, insbesondere mit dessen kennzeichnenden Merkmalen, wonach die Sterilisationseinrichtung mit wenigstens einem Verschlussorgan ausgestattet ist, welches dem Verschluss behälterfreier Aufnahmeöffnungen des Tragelementes während der Sterilisation dient.
Der wesentliche Vorteil der Erfindung liegt darin, dass durch das Verschlussorgan die behälterlosen Tragelemente in gleicher Weise durch die Sterilisationseinrichtung behandelt werden können, wie Tragelemente, deren Aufnahmeöffnungen mit Einzelbehältern besetzt sind. Insbesondere der auf die vergleichsweise kurze Verweildauer der Tragelemente in der Sterilisationseinrichtung sowie in der nachfolgenden Trocknungseinrichtung definierte Peroxidnebel verhält sich in seinem Strömungs- und Absetzungsverhalten dank des Verschlussorgans in nahezu identischer Weise wie bei der Sterilisation von mit Behältern besetzten Tragelementen. Eine prinzipiell mögliche Neujustage des Sterilisationsprozesses für unbestückte Tragelemente, wie beispielsweise eine Veränderung der Verweildauer, eine Anpassung des Peroxidnebels oder einer Anpassung der anschließenden Trocknung ist dank des Verschlussorgans nicht erforderlich. Die erfindungsgemäße Vorrichtung kann deshalb ohne Anpassung der Sterilisations- oder Vorzugsparameter auch mit behälterlosen Tragelementen betrieben werden.

Es ist vorgesehen, dass das Verschlussorgan unterhalb der zur sterilisierenden Tragelemente angeordnet ist.

Im Gegensatz zu einer Simulation einsitzender Behälter, welche durch ihren Tragrand auf der Oberseite der Tragelemente aufsitzen hat die Erfindung insoweit einen neuen Weg eingeschlagen, als dass sie das Verschlussorgan von unten an das Tragelemente heranführt und so den Verschluss der Aufnahmeöffnungen von der Unterseite des Tragelementes her realisiert. Diese hat den wesentlichen Vorteil, dass die Oberseite für den Auftrag des Sterilisationsnebels voll zugänglich ist und das Verschlussorgan nicht passgenau in die Aufnahmeöffnung eintauchen muss, stattdessen kann das Verschlussorgan beispielsweise an die Unterseite des Tragelementes angelegt werden, womit eine weniger exakte Positionierung von Verschlussorgan und Tragelement zueinander gewährleistet sein muss als es bei einer Simulation eines einsitzenden Behälters durch das Verschlussorgan notwendig wäre.

Sodann ist vorgesehen, dass das Verschlussorgan Teil der Anhebevorrichtung für die Einzelbehälter ist.

Die Erfindung macht sich die technischen Gegebenheiten der Sterilisationseinrichtung zu Nutze, indem sie das Verschlussorgan in die Anhebevorrichtung für die Einzelbehälter integriert. Dies hat vielerlei Vorteile. Beispielsweise sind in diesem Bereich Antriebe und sonstige technische Einrichtungen vorhanden, die auch zur Steuerung des Verschlussorgans genutzt werden können.

Vorgesehen ist ferner, dass die Steuerung bei in den Aufnahmeöffnungen einsitzenden Einzelbehältern eine Anhebebewegung der Anhebevorrichtung und bei behälterlosen Aufnahmeöffnungen eine Verschlussbewegung des Verschlussorgans induziert.

In vorteilhafter Weise werden gemäß dieser Ausführungsform die vorhandenen Antriebe sowohl für die Anhebevorrichtung wie auch für das Verschlussorgan genutzt, um je nach Beladungszustand des Tragelementes entweder eine Verschlussbewegung des Verschlussorgans oder aber eine Anhebebewegung der Anhebevorrichtung zum spaltbildenden Ausheben der Behälter aus den Tragelement-Aufnahmeöffnungen zu induzieren.

Vorgesehen ist ferner, dass die Anhebevorrichtung zumindest einen Hebeteller zum Anheben der Einzelbehälter aufweist und dass dieser Hebeteller das Verschlussorgan bildet.

In vorteilhafter Weise nutzt die Erfindung gemäß dieser Ausführungsform die Anhebeeinrichtung bzw. deren Hebeorgane selbst in einer Doppelfunktion, einerseits wird das Hebeorgan - hier ein Hebeteller - zum Anheben der Einzelbehälter genutzt. Sind die Tragelemente behälterfrei, dient das Hebeorgan als Verschlussorgan. Dieser Doppelnutzen des Verschlussorgans hat den wesentlichen Vorteil, dass lediglich die Steuerung und der Antrieb der Anhebevorrichtung angepasst werden müssen, um eine wirksame Sterilisation der Tragelemente in behälterlosem Zustand zu erreichen.

Vorgesehen ist ferner, dass mehrere Hebeteller vorgesehen und zu separat steuerbare Funktionsgruppen bilden, insbesondere wenn die Steuerung die in Funktionsgruppen zusammengefassten Hebeteller in Abhängigkeit freier oder mit Behältern bestückter Aufnahmeöffnungen in eine Anhebestellung oder eine Verschlussstellung bewegt, wobei bevorzugt ist, bei einer mehrere Tragelemente gleichzeitig sterilisierenden Sterilisationseinrichtung jedem in der Sterilisationseinrichtung befindlichen Tragelement wenigstens eine Funktionsgruppe zugeordnet ist.

Der wesentliche Vorteil hier liegt darin, dass die Sterilisationseinrichtung von mehreren Tragelementen durchlaufen wird und die Anhebevorrichtung durch entsprechende Ausgestaltung sowohl behälterfreie Tragelemente verschließen als auch Behälter in bestückten Tragelementen zur Sterilisation anheben kann.

Weitere Vorteile der Erfindung sowie eine besseres Verständnis derselben folgt aus der nachfolgenden Beschreibung eines Ausführungsbeispiels. Es zeigen:
- Fig. 1: eine vereinfachte Darstellung der erfindungsgemäße Vorrichtung,
- Fig. 1A: eine Darstellung eines Einzelbechers
- Fig. 2: die Aufsicht auf ein Tragelement,
- Fig. 3: eine entlang der Vorzugsrichtung geschnittene, vereinfachte Darstellung der Sterilisationseinrichtung,
- Fig. 4: die Darstellung gemäß Figur 3 mit Anhebevorrichtung in Verschlussposition,
- Fig. 5: die Darstellung gemäß Figur 3 mit Anhebevorrichtung in Verschluss- und Anhebeposition.

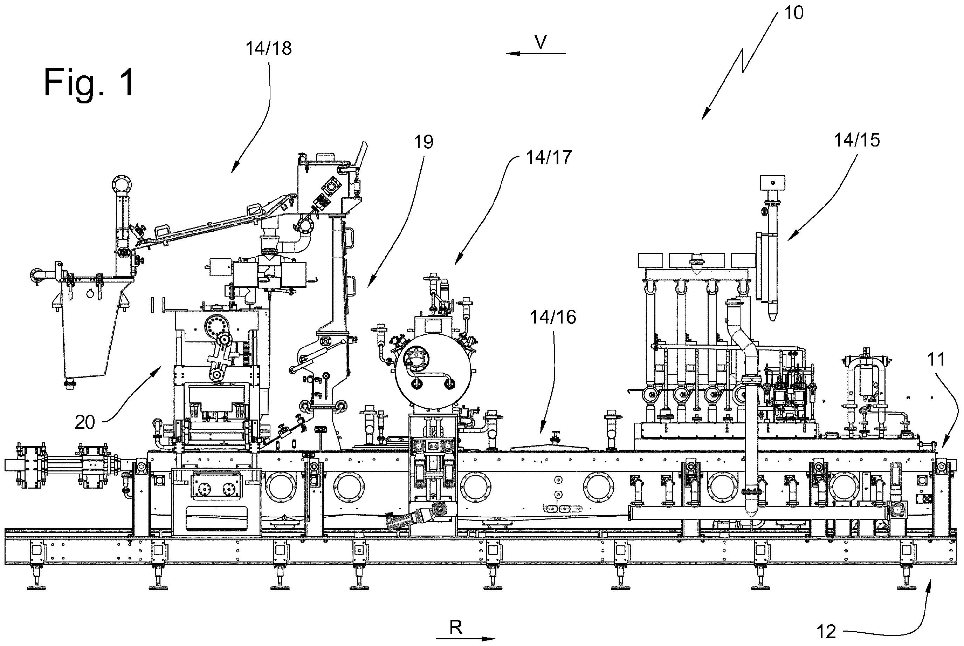

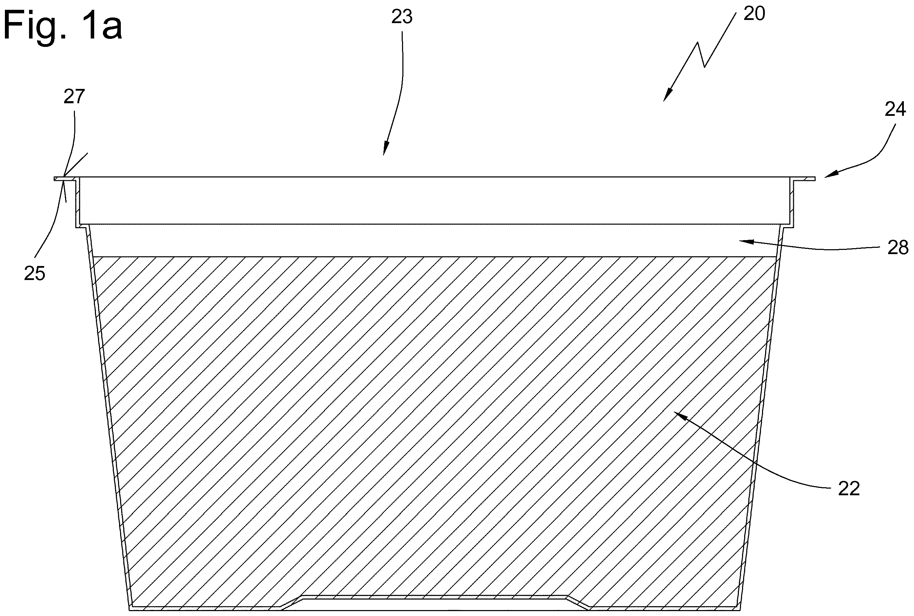

In den Figuren ist eine erfindungsgemäße Vorrichtung insgesamt mit der Bezugsziffer 10 bezeichnet und in Figur 1 in ihrer Gesamtheit dargestellt. Die Vorrichtung verfügt über ein Obertrum 11 sowie ein Untertrum 12, welche durch zwei endseitige, nicht dargestellte Seitentrümmer miteinander verbunden sind. Tragelemente 13, auch als Zellenbretter bezeichnet durchlaufen die Trümmer 11 und 12 umlaufend und endlos. Zu deren Vortrieb wird im hier dargestellten Ausführungsbeispiel eine in Figur 1 nicht sichtbare, endlos umlaufende Antriebskette verwendet. Arbeitsstationen 14 befinden sich oberhalb des Obertrums 11 in Vorschubrichtung V des Obertrums 11 hintereinander angeordnet.

Ausgehend von dem in Figur 1 rechts beginnenden und links endenden Obertrum befindet sich zunächst eine Bechereinsetzeinrichtung 15, eine Sterilisationseinrichtung 16, eine Befülleinrichtung 17 sowie eine Siegeleinrichtung 18. Die Siegeleinrichtung 18 unterteilt sich in eine Siegelfolienzuführung 19 und die eigentliche, die Siegelfolie auf den Einzelbehältern 20 aufbringende Siegelstation 21.

Ein am Anfang des Obertrums 11 einlaufendes Tragelement 13 bewegt sich entlang der Vorschubrichtung V durch das Obertrum 11. Es wird zunächst in der Bechereinsetzeinrichtung 15 mit Einzelbehältern 20 bestückt, gelangt dann zur Sterilisation des Tragelementes 13 sowie der eingesetzten Becher 20 in die Sterilisationseinrichtung 16. Bei einem weiteren Vorschub in Richtung Befülleinrichtung 17 wird das Tragelement 13 nebst eingesetzten Einzelbehältern 20 getrocknet. Sodann werden die Einzelbehälter 20 in der Befülleinrichtung 17 mit einem flüssigen bis pastösen Lebensmittel, hier konkret mit einem gering säurehaltigen Lebensmittel befüllt. Der unbefüllte Kopfraum 28 des Einzelbehälters 20 wird mit Stickstoff gespült, um den Sauerstoffgehalt auf das gewünschte Maß zu senken und wird dann in Vorschubrichtung V zur Siegeleinrichtung 18 überführt. In der Siegelstation 21 wird über entsprechende Siegelköpfe die Siegelfolie über der Behälteröffnung festgelegt. Im Anschluss daran werden die Behälter 20 dem Tragelement 13 entnommen und das Tragelement 13 wechselt am Ende des Obertrums 11 über ein nicht dargestelltes Seitentrum in das Untertrum 12. Das Untertrum 12 durchläuft das Tragelement 13 in Rückführrichtung R, bis es am Ende des Untertrum 12 wieder in das Obertrum 11 überführt wird.

Figur 1A zeigt einen Einzelbecher 20 in Schnittdarstellung. Bei dem Becher 20 handelt es sich um einen sogenannten Einzelbecher, welcher vorgefertigt angeliefert und zum Einsetzen in die Tragelemente 13 einem Vorrat entnommen wird. Die in der erfindungsgemäßen Vorrichtung eingesetzten Einzelbecher 20 sind insoweit im Gegensatz zu tiefgezogenen Behältern zu sehen. Zwar werden auch solche häufig zur Befüllung mit Nahrungsmitteln eingesetzt. Diese Tiefziehbehälter werden jedoch unmittelbar vor dem Befüllprozess geformt und erst nach der Versiegelung der Behälter voneinander getrennt.

Die verwendete Becherart hat im Hinblick auf den Abfüllprozess erhebliche Unterschiede zur Folge. Bei Tiefziehbechern sind die Tragelemente 13 von der Tiefziehfolie vollständig abgedeckt, wohingegen bei Einzelbechern die Tragelementoberfläche über die Zwischenräume zwischen den Einzelbechern 20 offen zugänglich ist. In Folge dessen ergibt sich bei der Verwendung von Tiefziehbehältern die Notwendigkeit der Tragelementsterilisation nicht im gleichen Maße.

Der in Figur 1A dargestellte Einzelbecher ist mit einem Lebensmittelprodukt 22 befüllt und bildet im Bereich der Becheröffnung 23 einen umlaufenden Tragrand 24 auf. Die Unterseite 25 des Tragrandes dient der Auflage auf dem Tragelement 13, um ein Durchrutschen durch die Aufnahmeöffnung 26 zu verhindern. Die Oberseite 27 des Tragrandes 24 bildet die Kontaktfläche zwischen Becher 20 und einer Siegelfolie.

Oberhalb des Lebensmittels 22 bis zum Tragrand 24 bzw. bei geschlossenem Becher 20 bis zur Siegelfolie befindet sich der sogenannte Kopfraum 28 des Bechers 20, welcher grundsätzlich gasgefüllt ist. Insbesondere bei wenig sauren, leicht verderblichen Lebensmitteln wird hier zur Minimierung des Sauerstoffgehaltes in der Regel Stickstoff eingeblasen.

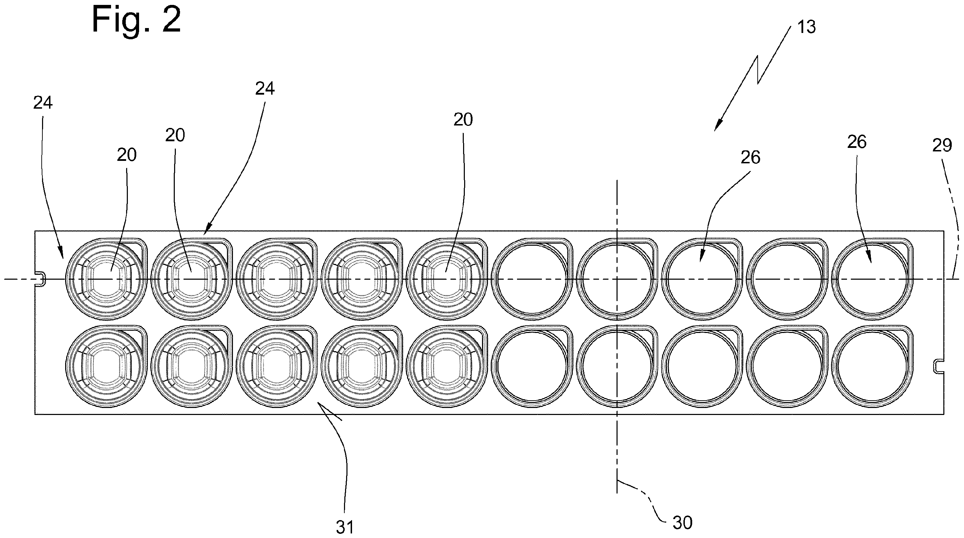

Figur 2 zeigt ein Tragelement 13, welches Aufnahmeöffnungen 26 ausbildet, die zur Illustration teilweise mit Einzelbechern 20 besetzt sind. Die Aufnahmeöffnungen 26 bilden in Vorzugsrichtung V gesehen hintereinander Behälterreihen 29 und sind in vorzugsrichtungsparallelen Bahnen 30 nebeneinander angeordnet. Wie Figur 2 zu entnehmen ist, sitzen die Becher 20 in den Aufnahmeöffnungen 26 ein, wobei der Tragrand 24 der Becher auf der Oberseite 31 des Tragelementes 13 aufliegt. Der Tragrand 24 eines jeden Bechers 20 deckt somit einen Oberflächenbereich der Oberseite 31 ab.

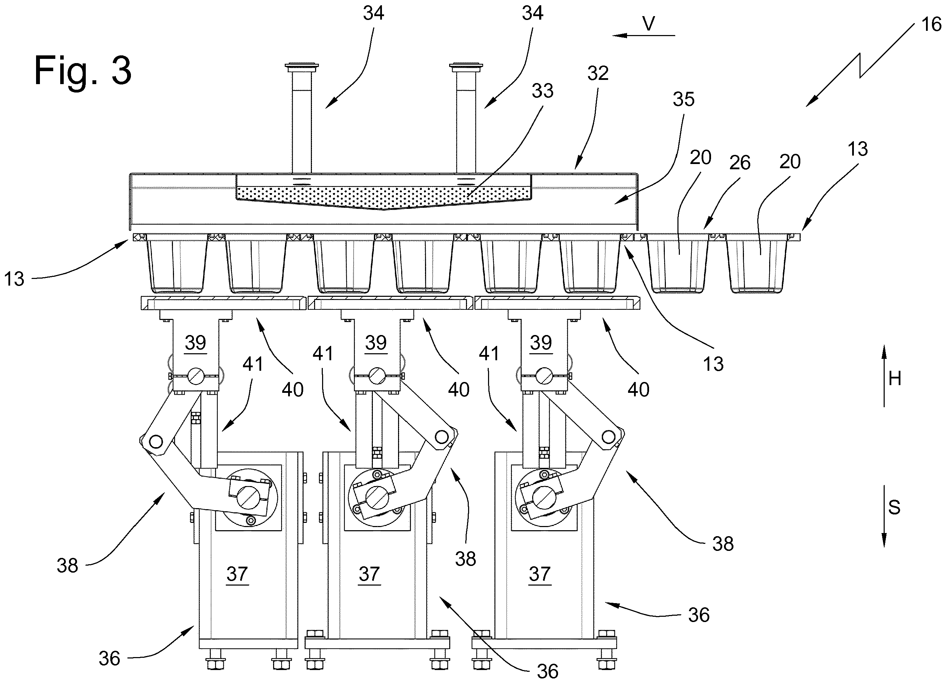

Die Figuren 3 bis 5 zeigen eine schematische Darstellung der Sterilisationseinrichtung 16. Dargestellt sind nur die für die Erfindung wesentlichen Bauteile, andere Bauteile wurden in der Darstellung der besseren Übersicht halber weggelassen.

Die Tragelemente 13 durchlaufen die Sterilisationseinrichtung 16 in Vorzugsrichtung V. In Figur 3 sind sämtliche Tragelemente 13 mit Einzelbechern 20 bestückt, die in den Aufnahmeöffnungen 26 einsitzen.

Der Sterilisationsprozess findet unter einer Haube 32 statt, unter welcher ein Diffusor 33 angeordnet ist, welcher einen über Zuleitungen 34 eingebrachten Peroxidnebel im Sterilisationsraum 35 verteilt. Unterhalb der Tragelemente 13 im Bereich der Sterilisationshaube 32 sind drei Anhebevorrichtungen 36 angeordnet. Dabei ist jede der Anhebevorrichtungen 36 unterhalb genau eines Tragelementes 13 befindlich und verfügt zunächst über eine Antriebseinheit 37. Die Antriebseinheit 37 wirkt auf einen Betätigungsarm 38, welcher dem Anheben oder Absenken eines Stempels 39 dient. Der Stempel 39 hält auf seiner dem Tragelement 13 zugewandten Seite einen Hebeteller 40. Jeder Stempel 39 wird in einer Schiene 41 geführt, welche eine vertikale Hubbewegung H und Senkbewegung S sicherstellt.

In Figur 3 befinden sich die Hebeteller 40 in einer abgesenkten Stellung, welche den Vorzug der Zellenbretter 13 ermöglicht. Figur 3 zeigt auch den üblichen Betrieb der Sterilisationseinrichtung 16. Im Regelbetrieb ist jedes Tragelement 13 unterhalb der Haube 32 mit Einzelbechern 20 besetzt. Vor dem Einlassen des Peroxidnebels in den Sterilisationsraum 35 erfahren die Hebeteller 40 eine Hubbewegung in Richtung H. diese führt zur Anlage der Hebeteller 40 an dem Boden der Einzelbecher 20, wobei eine fortgesetzte Hubbewegung die Becher 20 aus dem Tragelement 13 aushebt. Der Aushebevorgang führt zu einer Spaltbildung zwischen dem Tragrand 24 eines jeden Bechers 20 und der Oberseite 31 eines jeden Tragelementes 13. Der nunmehr eingeblasene Peroxidnebel benetzt nicht nur die Becherinnenflächen und die freien Oberflächenbereiche der Tragelemente 13 sondern erreicht auch den zwischen Tragrand 24 und Oberseite 31 bestehenden Spalt, so dass die Tragelemente 13 auch in ihren unterhalb des Tragrandes 24 gelegenen Bereichen sterilisiert werden. Nach der Sterilisation werden die Hebeteller durch eine Senkbewegung S wieder in ihre Ausgangsstellung zurückverfahren, wodurch die Becher 20 wieder in den Tragelementen 13 einsitzen. Es erfolgt dann ein Vorzugstakt, welcher in der Regel das in Vorzugsrichtung V vorne liegende Tragelemente zur nächsten Arbeitsstation überführt. Mit dem erneuten Anheben der Hebeteller 40 beginnt sodann der nächst Sterilisationsvorgang der Vorrichtung 10.

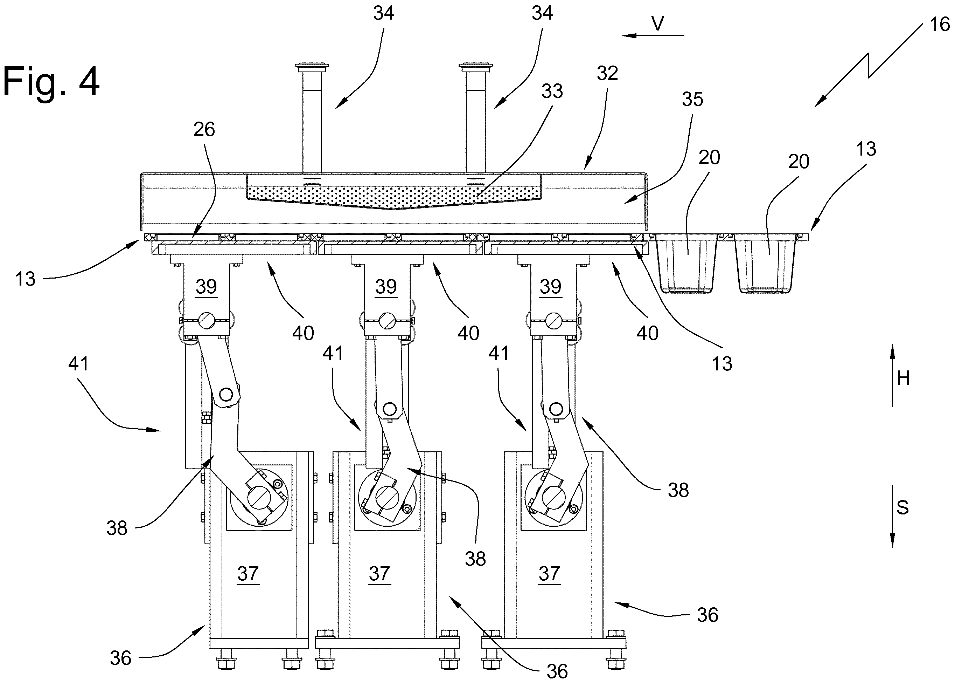

Figur 4 zeigt die Sterilisationseinrichtung 16 gemäß der Darstellung in Figur 3, wobei hier die unter der Sterilisationshaube 32 befindlichen Tragelemente becherlos sind. Die in Figur 4 dargestellte Situation kann beispielsweise beim Anlaufen der Vorrichtung 10 eintreten, wenn noch nicht mit Bechern 20 bestückte Tragelemente 13 die Vorrichtung 10 durchlaufen. Über geeignete Sensoren erfasst eine die Anhebevorrichtung 16 betreibende Steuerung die Belegung der Tragelemente 13 mit Bechern 20 und hat hier aufgrund der fehlenden Belegung die Hebeteller 40 über ihre Anhebeposition hinaus in eine die Aufnahmeöffnungen 26 verschließende Verschlussposition verfahren. In dieser Verschlussposition liegen die Hebeteller 40 an der Unterseite der Tragelemente 13 an und verschließen so die Aufnahmeöffnungen 26. Der eingeblasene Peroxidnebel kann sich ohne störende Einflüsse nicht besetzter Aufnahmeöffnungen 26 im Sterilisationsraum 35 verteilen und die Tragelementoberflächen benetzen. Vor dem nächsten Vorzugtakt werden die Hebeteller 40 wieder in ihre abgesenkte Position verfahren, wodurch dann ein freier Vorzug der Tragelemente 13 gewährleistet ist.

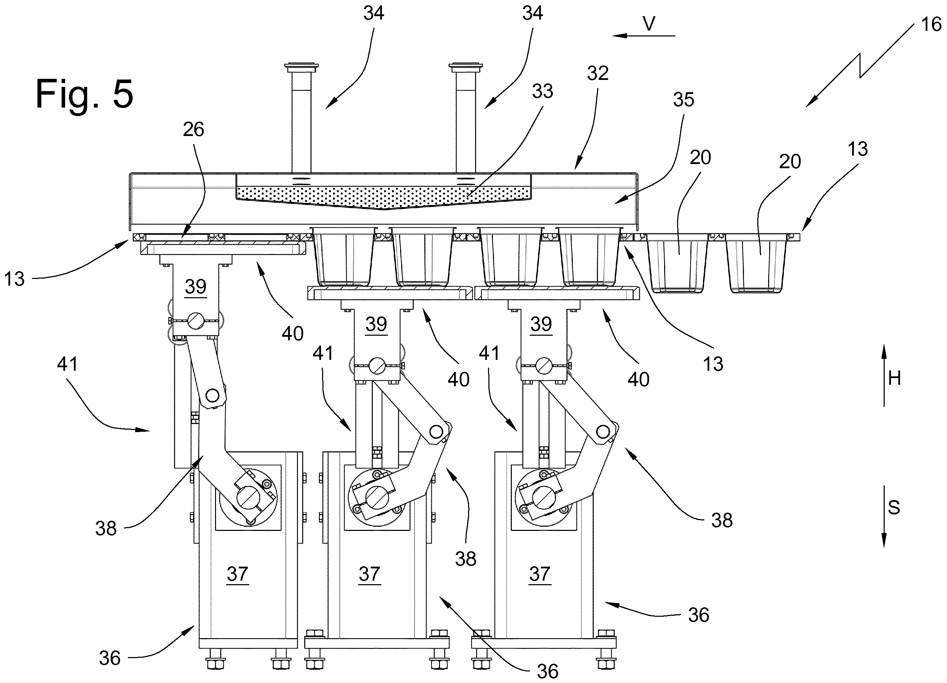

Eine wiederum abweichende Betriebssituation der Sterilisationseinrichtung 16 ist in Figur 5 gezeigt. Eines der unterhalb der Sterilisationshaube 32 angeordneten Tragelemente 13 ist becherlos gehalten, zwei andere sind mit Einzelbechern 20 bestückt. Dargestellt ist hier, wie die Aufnahmeöffnungen 26 des behälterlosen Tragelementes 13 durch den Hebeteller 40 verschlossen sind. Hierzu hat die Steuerung der Anhebevorrichtung 16 bei dem in Vorzugsrichtung V vorne liegenden Antrieb 37 eine Verschlussbewegung in Richtung H aufgezwungen. Den beiden anderen Antrieben wurde hingegen lediglich eine Hebebewegung induziert, so dass die in den Tragelementen 13 einsitzenden Becher 20 für den anstehenden Sterilisationsprozess spaltbildend angehoben wurden. Nach Abschluss des Sterilisationsvorganges werden auch in dieser Betriebssituation die Hebeteller 40 wieder in ihre Ausgangsstellung abgesenkt, um den Vorzug der Tragelemente 13 in Vorzugsrichtung V zu ermöglichen.

Insbesondere der Darstellung gemäß Figur 5 kann man entnehmen, dass die einzelnen Anhebevorrichtungen 36 unabhängig gesteuert werden können, so dass auf die jeweilige Befüllsituation der Tragelemente 13 mit Bechern 20 individuell reagiert werden kann. Insoweit sind bei der vorliegenden Ausführungsform die Anhebevorrichtungen 36 separat steuerbare Funktionsgruppen.

Zusammenfassend wurde eine vorteilhafte Sterilisationseinrichtung 16 mit Anhebevorrichtung 36 für eine Vorrichtung 10 zum Abfüllen von Lebensmitteln gezeigt, welche einen sicheren Sterilisationsprozess becherloser Tragelemente 13 gewährleistet. Dabei wird die Anhebevorrichtung 36 genutzt, um Verschlussorgane zu steuern, welche die Aufnahmeöffnungen 26 der Tragelemente 13 von unten her verschließen. In besonders vorteilhafter Weise werden die ohnehin vorhandenen Hebeteller 40 als Verschlussorgan genutzt. Bei einer Sterilisationseinrichtung 16, welche mehrere Tragelemente 13 gleichzeitig sterilisiert ist vorgesehen, mehrere Anhebevorrichtungen 36 einzusetzen, wobei jedem Tragelement 13 eine Anhebevorrichtung 36 zugeordnet ist. Die separate Ansteuerung der Anhebevorrichtungen 36 ermöglicht es sodann, die jeweilige Befüllsituation der Tragelemente 13 mit Bechern 20 zu berücksichtigen.

### Bezugszeichenliste

- 10: Vorrichtung
- 11: Obertrum
- 12: Untertrum
- 13: Tragelement / Zellenbrett
- 14: Arbeitsstation
- 15: Bechereinsetzeinrichtung
- 16: Sterilisationseinrichtung
- 17: Befülleinrichtung
- 18: Siegeleinrichtung
- 19: Siegelfolienzuführung
- 20: Einzelbehälter / Einzelbecher
- 21: Siegelstation
- 22: Lebensmittel
- 23: Becheröffnung
- 24: Tragrand
- 25: Unterseite von 24
- 26: Aufnahmeöffnung
- 27: Oberseite von 24
- 28: Kopfraum von 20
- 29: Reihen
- 30: Bahn
- 31: Oberseite von 13
- 32: Haube
- 33: Diffusor
- 34: Zuleitung
- 35: Sterilisationsraum
- 36: Anhebevorrichtung
- 37: Antriebseinheit
- 38: Betätigungsarm
- 39: Stempel
- 40: Hebeteller
- 41: Schiene

- H: Hubbewegung
- S: Senkbewegung
- R: Rückführrichtung
- V: Vorschubrichtung

## Patentansprüche

1. Vorrichtung zum Abfüllen von flüssigen bis pastösen Lebensmitteln in vorgefertigte Einzelbehälter, die einen Tragrand aufweisen,
- mit Tragelementen, die mittels eines Antriebs endlos durch die Vorrichtung geführt sind, Aufnahmeöffnungen für die Einzelbehälter ausbilden und eine der Schwerkraft abgewandte Oberseite aufweisen, auf welcher der Tragrand der in den Aufnahmeöffnungen einsitzenden Einzelbehälter aufzuliegen in der Lage ist,
- mit Arbeitsstationen, in deren Wirkbereich die Tragelemente auf ihrem endlosen Umlauf in der Vorrichtung gelangen,
- mit wenigstens einer als Sterilisationseinrichtung ausgebildeten Arbeitsstation, welche der Entkeimung von Tragelementen und Einzelbehältern dient,
- mit einer Anhebevorrichtung als Teil der Sterilisationseinrichtung, welche in den Aufnahmeöffnungen einsitzende und mit dem Tragrand auf der Oberseite aufliegende Einzelbehälter zur Sterilisation spaltbildend anzuheben in der Lage ist,
- mit einer Steuerung, die zumindest der Steuerung der Anhebevorrichtung dient,
**dadurch gekennzeichnet, dass** die Sterilisationseinrichtung mit wenigstens einem Verschlussorgan ausgestattet ist, welches dem Verschluss behälterfreier Aufnahmeöffnungen des Tragelementes während der Sterilisation dient.

2. Vorrichtung nach Anspruch 1, **dadurch gekennzeichnet, dass** das Verschlussorgan unterhalb der zur sterilisierenden Tragelemente angeordnet ist.

3. Vorrichtung nach Anspruch 1 oder 2, **dadurch gekennzeichnet, dass** das Verschlussorgan Teil der Anhebevorrichtung für die Einzelbehälter ist.

4. Vorrichtung nach Anspruch 2 oder 3, **dadurch gekennzeichnet, dass** die Steuerung bei in den Aufnahmeöffnungen einsitzenden Einzelbehältern eine Anhebebewegung der Anhebevorrichtung und bei behälterlosen Aufnahmeöffnungen eine Verschlussbewegung des Verschlussorgans induziert.

5. Vorrichtung nach einem der Ansprüche 3 oder 4, **dadurch gekennzeichnet, dass** die Anhebevorrichtung zumindest einen Hebeteller zum Anheben der Einzelbehälter aufweist und dass dieser Hebeteller das Verschlussorgan bildet.

6. Vorrichtung nach Anspruch 5, **dadurch gekennzeichnet, dass** mehrere Hebeteller vorgesehen sind und separat steuerbare Funktionsgruppen bilden.

7. Vorrichtung nach Anspruch 6, **dadurch gekennzeichnet, dass** die Steuerung die in einer gemeinsamen Funktionsgruppen zusammengefassten Hebeteller in Abhängigkeit freier oder mit Behältern bestückter Aufnahmeöffnungen in eine Anhebestellung oder eine Verschlussstellung bewegt.

8. Vorrichtung nach Anspruch 7, **dadurch gekennzeichnet, dass** bei einer mehrere Tragelemente gleichzeitig sterilisierenden Sterilisationseinrichtung jedem in der Sterilisationseinrichtung befindlichen Tragelement wenigstens eine Funktionsgruppe zugeordnet ist.

## Claims

1. Device for filling liquid to pasty foodstuffs into premanufactured individual containers which have a support rim,
- comprising carrier elements which are continuously guided through the device by means of a drive and which form receiving openings for the individual containers and have an upper side oriented away from the force of gravity, on which the support rim of the individual containers seated in the receiving openings is able to bear,
- comprising operating stations, into the operating range of which the carrier elements pass as they revolve continuously in the device,
- comprising at least one operating station configured as a sterilisation unit which serves for disinfecting the carrier elements and the individual containers,
- comprising a lifting means as part of the sterilisation unit, which lifting means is able to lift individual containers, which are seated in the receiving openings and bear with the support rim against the upper side, so as to form a gap for sterilisation purposes,
- comprising a controller which serves at least to control the lifting means,
**characterised in that** the sterilisation unit is equipped with at least one closure element which serves to close container-free receiving openings of the carrier element during the sterilisation.

2. Device according to claim 1, **characterised in that** the closure element is arranged below the carrier elements to be sterilised.

3. Device according to claim 1 or 2, **characterised in that** the closure element is part of the lifting means for the individual containers.

4. Device according to claim 2 or 3, **characterised in that** the controller induces a lifting movement of the lifting means in the case of individual containers being seated in the receiving openings and induces a closing movement of the closure element in the case of container-free receiving openings.

5. Device according to one of claims 3 or 4, **characterised in that** the lifting means has at least one lifting plate for lifting the individual containers, and **in that** said lifting plate forms the closure element.

6. Device according to claim 5, **characterised in that** a plurality of lifting plates are provided and form separately controllable functional groups.

7. Device according to claim 6, **characterised in that** the controller moves the lifting plates pooled into a common functional group into a lifting position or a closing position depending on whether the receiving openings are empty or are occupied by containers.

8. Device according to claim 7, **characterised in that**, in the case of a sterilisation unit which sterilises a plurality of carrier elements simultaneously, at least one functional group is assigned to each carrier element located in the sterilisation unit.

## Revendications

1. Dispositif pour le remplissage de récipients individuels préfabriqués de produits alimentaires liquides à pâteux, qui ont un bord de support,
- avec des éléments de support, qui sont guidés au moyen d'un entraînement sans fin à travers le dispositif, qui forment des ouvertures de réception pour les récipients individuels et comportent une partie supérieure éloignée du centre de gravité, sur laquelle le bord de support des récipients individuels placés dans les ouvertures de réception peut reposer,
- avec des stations de travail, dont les éléments de support atteignent leur portée effective sur leur circulation sans fin dans le dispositif,
- avec au moins une station de travail conçue comme un dispositif de stérilisation qui sert pour la stérilisation des éléments de support et des récipients individuels,
- avec un dispositif de levage faisant partie du dispositif de stérilisation, qui est capable de lever les récipients individuels reposant sur le côté supérieur avec le bord de support et placés dans les ouvertures de réception, pour stérilisation, sous forme de fente.
- avec une commande, qui sert au moins de commande du dispositif de levage,
**caractérisé en ce que** le dispositif de stérilisation est équipé d'au moins un organe de fermeture qui sert à sceller les ouvertures de réception, sans récipient, de l'élément de support pendant la stérilisation.

2. Dispositif selon la revendication 1, **caractérisé en ce que** l'organe de fermeture est disposé en dessous des éléments de support à stériliser.

3. Dispositif selon la revendication 1 ou 2, **caractérisé en ce que** l'organe de fermeture est partie du dispositif de levage pour les récipients individuels.

4. Dispositif selon la revendication 2 ou 3, **caractérisé en ce que** la commande entraine, lorsque les récipients individuels sont dans les ouvertures de réception, un mouvement de levage du dispositif de levage, et lorsque les ouvertures de réception sont sans récipient, un mouvement de fermeture de l'organe de fermeture.

5. Dispositif selon l'une des revendications 3 ou 4, **caractérisé en ce que** le dispositif de levage comporte au moins une plaque de levage pour lever les récipients individuels et **en ce que** cette plaque de levage forme l'organe de fermeture.

6. Dispositif selon la revendication 5, **caractérisé en ce que** plusieurs plaques de levage sont prévues et forment des groupes de fonction qui peuvent être commandés séparément.

7. Dispositif selon la revendication 6, **caractérisé en ce que** la commande bouge les plaques de levage regroupées un groupe de fonction commun, en fonction des ouvertures de réception sans ou avec récipients, dans une position de levage ou position de fermeture.

8. Dispositif selon la revendication 7, **caractérisé en ce que**, lorsque le dispositif de stérilisation stérilise en même temps plusieurs éléments de support, au moins un groupe de fonction est associé à chaque élément de support placé dans le dispositif de stérilisation.
